# EUROPEAN PATENT SPECIFICATION

(11) **EP 2 910 131 B1**
(45) Date of publication and mention of the grant of the patent: **27.11.2019**
(21) Application number: 14826277.7
(22) Date of filing: 09.07.2014
(51) Int. Cl.: A23L 33/10, A23F 5/40, A23G 3/36, A23G 3/42, A23G 3/48, A23L 2/38, A23L 2/52, A23L 33/105, A61K 36/74

(54) **ANTI-FATIGUE COMPOSITION AND USE THEREOF**
ZUSAMMENSETZUNG ZUR MÜDIGKEITSBEKÄMPFUNG UND VERWENDUNG DAVON
COMPOSITION ANTI-FATIGUE ET SON UTILISATION

(30) Priority: 15.07.2013 CN 201310295767; 13.11.2013 US 201361903783 P; 22.05.2014 CN 201410218857
(43) Date of publication of application: 26.08.2015
(73) Proprietor: Chengzhi Life Science., Ltd., Hebei Province (CN); Beijing Chengzhi Life Science Co., Ltd., Beijing 10084 (CN); Bioenergy Life Science, Inc., Ham Lake, MN 55304 (US)
(72) Inventor: WU, Dan, Beijing 100083 (CN); CEN, Yu, Beijing 100084 (CN); XUE, Yongquan, Blaine, Minnesota 55449 (US); GAO, Runxiang, Beijing 102208 (CN)
(74) Representative: Kolster Oy Ab
(86) International application number: PCT/CN2014/081917
(87) International publication number: WO 2015/007177

(56) References cited:
- CN-A- 101 756 206
- CN-A- 101 919 801
- JP-A- 2005 336 176
- US-A1- 2005 048 136
- US-A1- 2006 105 965
- US-A1- 2006 105 965
- US-A1- 2012 100 120
- US-A1- 2012 100 120
- HERRICK J ET AL: "D-ribose - An additive with caffeine", MEDICAL HYPOTHESES, EDEN PRESS, PENRITH, US, vol. 72, no. 5, 1 May 2009 (2009-05-01), pages 499-500, XP026032879, ISSN: 0306-9877, DOI: 10.1016/J.MEHY.2008.12.038 [retrieved on 2009-02-14]
- JIM HERRICK ET AL.: 'D-ribose - An additive with caffeine.' MEDICAL HYPOTHESES vol. 72, 31 December 2009, pages 499 - 500, XP055252311

## Description

### Technical field

The present invention relates to the field of food science, particularly, to a composition containing ribose and caffeine as effective ingredients, which has a sustainable anti-fatigue effect and helps the body restore the level of high-energy phosphate substance.

### Background Art

There are currently a number of functional products for anti-fatigue on the market, such as Red Bull, Qi Li, LiBaoJian (Lipovitan D) and the like, and the use of such products can quickly relieve the sense of fatigue from the body, refresh mind and keep excitement in spirit. Most of these products are in the form of beverage and generally contains caffeine with the content of about 15-100 mg based on 100 ml beverage, which has a role in excitement of central nerve.

As the pace of life is accelerated and the pressure for work and life increases, the varieties of food containing caffeine for anti-fatigue are increasing. The tracking analysis for FMCG (Fast-Moving Consumer Goods) market shows that a demand for various beverage products grows rapidly in recent years. More and more people try to achieve the purpose for anti-fatigue and refreshment relying on a variety of beverages containing caffeine. After applying such caffeinated products, the excitement in spirit can be obtained in a short time. However, when a certain amount of such caffeinated products have been drunk, the excitement does not further increase with the increased amount of drinking. On the contrary, when a large amount of these products are taken, it will make the body feel more tired, even result in deep fatigue. This is closely related to the action mechanism of caffeine. Caffeine is a central nervous system stimulant that can temporarily drive sleepiness away and recover energy. When the body feels tired, the amount of ATP consumed inside a body is much higher than that of ATP synthesized in the body, with decomposition of a large amount of ADP and AMP, which results in the increase in the adenosine content in blood. Adenosine may binds to the adenosine receptor in the brain of the body, making the body feel sleepy and tired. Caffeine mainly acts on the adenosine receptor in the brain of the body, inhibiting the binding of the receptor to adenosinehereby signals of tired cannot be sent. Therefore, caffeine has the effect of exciting the central nerve in a short period of time, during which the body does not feel tired, however, this effect does not fundamentally improve the state of fatigue in the body, and the physiological fatigue still exists. Since the amount of adenosine receptor in human body is definite, the excitement degree will not continuously increase with increasing in the amountof drunk caffeine. The human body will appear more obviously tired when consuming a large amount of coffee or caffeinated food. And the more one feel tired, the more coffee or food containing caffeine will be consumed. When the consumption of such foods reaches a certain limit, even if the consumption further increases, not only the effect of refreshment will not appear, but also some deep fatigue symptoms such as headache, weakness, etc. will be caused. This shows that although drinking coffee or caffeinated beverages has certain effect on relieving fatigue in a short time, the body's fatigue still exists. If the state of fatigue cannot be really improved, continuous consumption of lots of caffeinated foods will result in long-term fatigue in the body, and the human organism will be damaged when being chronically under the condition.

Caffeine is a stimulant for central nervous system, which has a high content in coffee. Coffee, a much-loved traditional drink in the West, has the effect of refreshing and reducing fatigue. Currently a lot of foods and drinks contain caffeine. Caffeine has multiple pharmacological effects, and there are many situations for obtained experimental results of caffeine due to great differences in dosages of caffeine used in many studies. At present the most direct evidence about the pharmacological effect of caffeine on motion comes from the research conducted by Davis et al. The research shows that the central role of caffeine on motor ability is mediated by adenosine receptor pathway. The mechanism is complex and may include: 1. enhancing the activity of sympathetic nervous system; 2. increasing discharge and/or synchronous contraction of motor units; 3. increasing the recruitment of skeletal muscle motor units; 4. reducing the subjective feeling for fatigue and pain resulting from movement. In addition, caffeine acts directly on different receptors on the sarcoplasmic reticulum and increases muscle tension through excitation-contraction coupling effect on the skeletal muscle, so as to regulate the contractions of skeletal muscle and cardiac muscle.

Ingesting a large amount of caffeine, usually more than 250 mg (equivalent to 2-3 cups of boiled coffee), in a short time, may result in hyperexcitability of central nervous system. Hyperexcitability behaviors caused by caffeine include: dysphoria, nervousness, excitement, insomnia, flushing, increasing-urine, gastrointestinal disorders, muscle twitch, mind wandering, irregular heartbeats, or tachycardia and restlessness, etc., which all will make the body feel discomfortable.

As a natural pentose, ribose is phosphorylated to ribose-5-phosphate upon entering the body under the effect of ribose kinases; it can also turn into phosphoribosyl pyrophosphate (PRPP) under the effect of phosphoribosyl pyrophosphate synthase. PRPP subsequently is involved in the pathways of de novo and remedial synthesis of nucleoside substances such as adenosine, inosine and ATP, etc., and it also plays a similar role in multiple organs of the body, including skeletal muscle and heart. The formation rate of ribose is slow through the pathway of pentose phosphate in human muscle due to the less enzyme content which is required in the reaction. Therefore, the supplement of exogenous ribose can skip the rate-limiting step of G6PDH in the pentose phosphate pathway, directly improve PRPP level, thus speed up the synthesis of purine nucleotide in heart and skeletal muscles and the restoration of ATP library of the body, and thereby relieve the body fatigue.

ATP, known as "energy currency" in cells, is a direct source of the energy required for all life activities of tissue cells in vivo. The storage and transfer of chemical energy, and the syntheses of protein, fat, sugar and nucleotides all require the participation of ATP, which can promote the repair and regeneration of various cells and tissues inside the body and enhance the metabolic activity of cells.

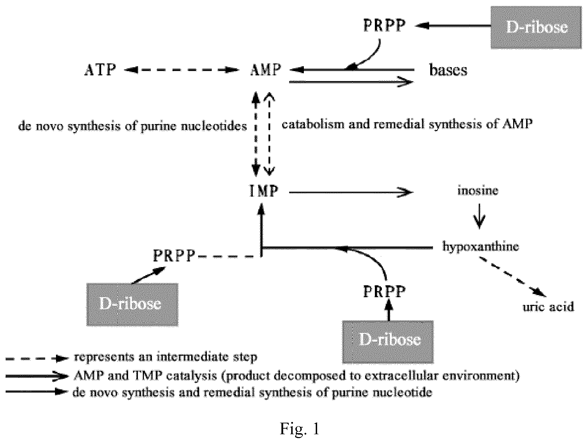

D-ribose is the starting material and limiting factor for the synthesis of ATP in the cells. D-ribose is involved in de novo and remedial synthesis metabolism of energy by the formation of PRPP (5-ribose phosphate-1-pyrophosphate) (Fig. 1). None of other substances could replace ribose to play an important regulatory role in nucleotide metabolism. Nucleotides including ATP are important energies for basic metabolism, and play an important role in synthesis of protein, glycogen and nucleic acids (RNA and DNA), together with nucleotide metabolism and energy conversion. The body will be short of energy if these important structural components are insufficient, such that protein cannot be further synthesized, making cells lose the replication ability.

Herrick et al. (Medical Hypotheses, 2009, Vol. 72, pp. 499-500) suggests that D-ribose could potentially aid in maintaining or potentially lowering extracellular adenoside concentrations, aid in the flux of intracellular calcium, aid in intracellular energy production, and potentially lessen the perceived "crash" state felt by many after caffeine consumption. However, no experimental data nor any composition comprising D-ribose and caffeine are disclosed.

US 2006/105965 discloses a composition for enhancing energy levels and/or decreasing stress in a mammal, comprising niacin bound chromium, D-ribose, a withanolide and at least one amine selected from phenylalanine, caffeine, taurine and glutamine.

US 2012/100120 discloses a nutraceutical supplement composition for enhancing physical performance, the composition comprising ribose, coenzyme Q10, a saccharide, ATP, caffeine and D-pinitol.

CN 101756206 discloses a health-care food comprising 0.01-99.999 percent by weight D-ribose and 0.001-99.999 percent by weight tea extract or tea powder, which may contain caffeine.

Currently, researches about the mechanism and the effect of caffeine in combination with ribose have not yet been reported.

### Summary of the Invention

The present invention is defined in the appended claims.

An object of the present invention is to provide a composition which has a sustainable anti-fatigue effect and helps the body restore the level of high-energy phosphate substances.

To achieve the object, the present invention provides a composition having a sustainable anti-fatigue effect, which contains ribose and caffeine as effective ingredients.

The composition containing ribose and caffeine of the present invention comprises the following components in parts by weight: 92.6-98 of ribose and 2-7.4 of coffee.

The composition containing ribose and caffeine of the present invention may further comprise an appropriate amount of one or more substances selected from the groups consisting of milk powder or fresh milk, amino acids, sugars, sugar alcohols, cereals, cocoa, chocolate, non-saccharide and sugar alcohols sweeteners, pectins, soluble dietary fibers, salts, other carbohydrates, vitamins, minerals, carbon dioxide or other food additives/excipients and the like.

Wherein, the amino acids comprise one or more of taurine, pyruvic acid, ketoglutaric acid, arginine, citrulline and the like; the sugars comprise one or more of glucose, sucrose, fructose and the like; the non-saccharide and sugar alcohols sweeteners comprise one or more of stevia, aspartame, sucralose, Momordica grosvenori, glycyrrhizin, beet sweetener, saccharin sodium and the like; the sugar alcohols comprise one or more of xylitol, erythritol, maltitol, isomalt, sorbitol and the like; the minerals comprise one or more elements of calcium, magnesium, iron, zinc and the like; the salts comprise one or more salts of sodium, potassium, phosphate and the like; said other carbohydrates comprise starch and/or insoluble dietary fiber and the like; said other food additives/excipients comprise one or more of essences, colorants, malic acid, citric acid, sodium citrate, sorbic acid, potassium sorbate, benzoic acid, sodium benzoate, edible gum base, sucrose ester, soy lecithin, sodium alginate and the like. The essences and colorants are synthetic or natural; and said caffeine is derived from cocoa, coffee, guarana, tea or chocolate and the like. Plant components can also be added into the product. The product may be in a form of solid, gel, semi-solid or liquid.

The following reference compositions of the present disclosure do not fall within the scope of the claims:
A reference composition containing ribose and caffeine of the present disclosure, which are respectively ribose and caffeine powder, comprises the following components in parts by weight: 1-99.9 ribose, 0.1-99 coffee, 0-40 white sugar and/or 0-20 fructose.

A reference composition containing ribose and caffeine of the present disclosure comprises the following components in parts by weight: 0.1-60 ribose, 0.01-100 coffee or aqueous extracts of coffee and 0-100 milk powder (skimmed milk powder) or fresh milk.

A reference composition containing ribose and caffeine of the present disclosure comprises the following components in parts by weight: 0.01-60 ribose, 0.0001-0.1 caffeine and 0-20 milk powder (skimmed milk powder) or fresh milk.

A reference composition containing ribose and caffeine of the present disclosure comprises the following components in parts by weight: 0.01-60 ribose, 0.0001-0.1 caffeine, 0-20 milk powder (skimmed milk powder) or fresh milk and 0-50 plant dairy.

A reference composition containing ribose and caffeine of the present disclosure comprises the following components in parts by weight: 0.01-60 ribose, 0.0001-0.1 caffeine and 0-20 amino acids.

A reference composition containing ribose and caffeine of the present disclosure comprises the following components in parts by weight: 0.1-60 ribose, 0.01-100 coffee or aqueous extracts of coffee and 0.01-20 milk powder or fresh milk.

A reference composition containing ribose and caffeine of the present disclosure comprises the following components in parts by weight: 0.01-60 ribose, 0.0001-0.1 caffeine and 0.01-20 milk powder or fresh milk.

A reference composition containing ribose and caffeine of the present disclosure comprises the following components in parts by weight: 0.01-60 ribose, 0.0001-0.1 caffeine and 0.01-20 amino acids.

A reference composition containing ribose and caffeine of the present disclosure comprises the following components in parts by weight: 0.1-60 ribose, 0.01-100 coffee or aqueous extract of coffee, 0-20 milk powder or fresh milk, 0-10 cocoa, 0.01-60 sugars, 0-15 sugar alcohols, 0-5 non-saccharide and sugar alcohols sweeteners, 0-10 pectin, 0-60 soluble dietary fibers, 0-60 of other carbohydrates, 0-0.5 vitamins, 0-5 essence, 0-50 edible gum base and / or 40-99 water.

A reference composition containing ribose and caffeine of the present disclosure comprises the following components in parts by weight: 0.01-60 ribose, 0.0001-0.1 caffeine, 0-100 chocolate, 0-20 milk powder or fresh milk, 0-100 cereal, 0.01-60 sugars, 0-5 non-saccharide sweeteners, 0-20 soluble dietary fibers, 0-0.5 vitamins, 0-10 citric acid, 0-2 sodium citrate, 0-3 sorbic acid or potassium sorbate, 0-2 benzoic acid or sodium benzoate, 0-5 carbon dioxide, 0-5 colorants, 0-5 essences and 1-99 water.

A reference composition containing ribose and caffeine of the present disclosure comprises the following components in parts by weight: 0.01-60 ribose, 0.0001-0.1 caffeine, 0.01- 20 amino acids, 0-5 minerals, 0-60 sugars, 0-5 non-saccharide sweeteners, 0-20 soluble dietary fibers, 0-10 citric acid, 0-3 sorbic acid or potassium sorbate, 0-2 benzoic acid or sodium benzoate, 0-0.5 carbon dioxide, 0-5 colorants, 0-5 essences and / or 50-99 water.

The aqueous extract of coffee used in the present invention is prepared by the following steps: carefully selecting coffee beans, followed by roasting, crushing, adding water into the beans to decoct the mixture obtained, extracting, filtering and concentrating to obtain the aqueous extract; or dissolving instant coffee in water and filtering to obtain aqueous solution.

The composition containing ribose and caffeine of the present invention is prepared by the method comprising the following steps: mixing various components proportionally to prepare a solid, semi-solid, gel or liquid product. The result by stability studies, it shows that the product obtained by adding ribose into coffee or caffeinated product has a stable quality. After storage at 40°C for 3 months, the product has no significant changes in taste, appearance and microflora, which means the product meets the relevant requirements for food.

The present invention further provides non-therapeutic use of a composition containing ribose and caffeine in the preparation of health care product which has a sustainable anti-fatigue effect and helps the body restore the level of high-energy phosphate substances.

The study of the present invention shows that, ribose and caffeine have different action mechanisms and effects in terms of anti-fatigue. When being administrated ribose, animals can quickly restore physical strength from the state of fatigue, so that it can be recovered to normal level in a short time. When being administered caffeine, animals can also restore some physical strength from the same state of fatigue in a short time, however, this level of restoration is temporary, and soon the animals will fall back into the state of fatigue.

The composition containing ribose and caffeine of the present invention can improve the body's energy and resist short-term and long-term fatigue in the body. The combination of caffeine and ribose can quickly refresh mind and excite spirit, while it can also supplement the energy required by the body and make the body full of physical strength. The composition is effective in improving the more tired condition and discomfort after a short time of excitement in the body, which is caused by the prior product containing caffeine as the main anti-fatigue ingredient, thus making the body maintain physical strength better. Experiments show that the combination of ribose and caffeine of the present invention can improve the body's energy overdraft and quickly restore the body's level of high-energy phosphate substances, so as to relieve the feeling of more fatigue caused by drinking a lot of coffee or caffeinated food. The composition of the present invention has a significant sustainable anti-fatigue effect.

### Brief Description of the Drawings

Figure 1 shows a process of ribose in de novo and remediation syntheses of ATP.

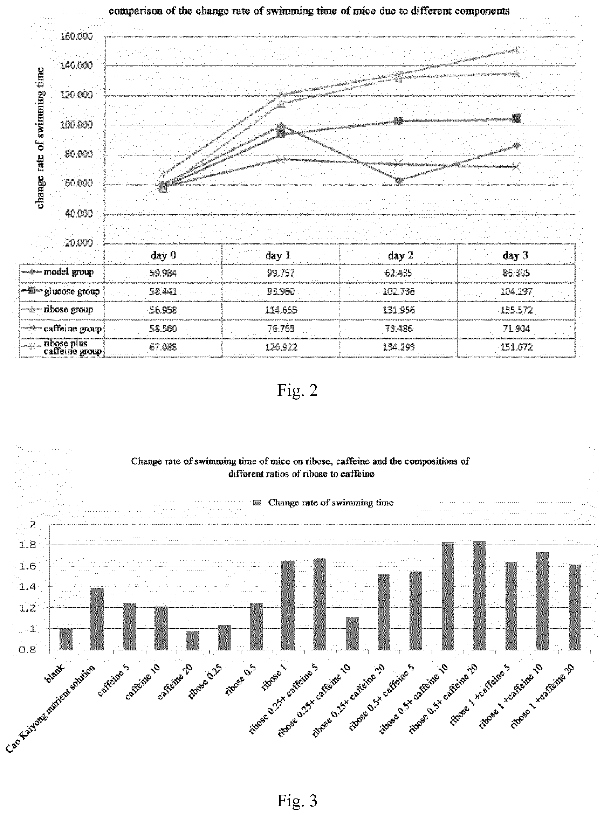

Figure 2 shows comparisons on the change rates of swimming time of mice due to different components according to Example 1 of the present invention. The test is performed by preparing mouse fatigue models to obtain fatigue mice, administrating ribose, caffeine and the composition of caffeine and ribose respectively to mice, and making the mice go back to swim until exhaustion. From that, it can be seen that these different components show different anti-fatigue effects for fatigue mice under continuous state of fatigue.
Figure 3 shows comparisons of the change rates of swimming time of mice due to different ratios of ribose to caffeine according to Example 2 of the present invention. The test is performed by preparing mouse fatigue models to obtain fatigue mice, administrating different doses of ribose, caffeine, and the compositions of different ratios of caffeine to ribose respectively to mice for 3 days, and on day 4, after 30 minutes since respectively administrating the corresponding components, making the mice swim until death. The comparisons of swimming time for each group and the model group are showed.

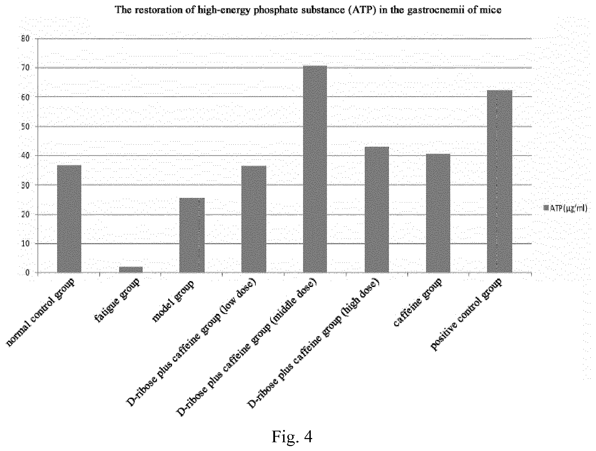

Figure 4 shows comparisons of the restorations of high-energy phosphate substance ATP in mice gastrocnemii among normal mice, model mice, the mice groups with taking ribose, caffeine, and caffeine plus ribose. After modeling, the gastrocnemii of 10 fatigue mice and 10 normal mice are immediately taken down, which form fatigue group and normal control group, respectively; the rest of the mice are randomly divided into six groups according to swimming time on day 1, namely the positive control group (Cao Kaiyong nutrient solution), model group, D-ribose plus caffeine group (high dose reference group) (ribose 1g + caffeine 20 mg/kg), D-ribose plus caffeine group (middle dose group) (ribose 0.5 g + caffeine 10 mg/kg), D-ribose plus caffeine (low dose reference group) (ribose 0.25 g + caffeine 5 mg/kg), and caffeine reference group (caffeine 20 mg/kg), with the administration volume of 10 ml/kg. After modeling, the intragastric administration is performed once daily for three days, after 30 minutes since the last administration, the gastrocnemii are taken down according to the method, and the contents of ATP, ADP, AMP and IMP in gastrocnemii are measured by high performance liquid chromatography. The table shows comparisons of ATP contents.

### Detailed Embodiments of the Invention

The following examples are used for illustrating the present invention but not intended to limit the scope of the present invention. Unless otherwise specified, the techniques used in the examples are conventional technical means known to those skilled in the art, and the materials used are commercially available.

### Example 1 Anti-fatigue test of ribose combined with caffeine

### 1. Establishment of fatigue mouse models

### 1.1 Experimental animal source

Experimental animals are ICR male mice with weight of 18-20 g, which are purchased from Beijing Vital River Laboratory Animal Technology Co., certificate of conformity No.: SCXK (Beijing) 2012-0001.

### 1.2 Weight loading swimming test of mice

A lead wire with a weight equivalent to 7.5% body weight of each mouse is fixed to the tail of the corresponding mouse, and the mice are placed into 30 cm×25 cm×25 cm water tanks with water tempreture of 30°C and water depth of 20 cm, with two mice per tank. The mice are made swim until the mice are fatigued (submerging in water for 4 seconds), after rest for 20 minutes, then the mice are made swim again until the mice are fatigued, and the times of the two swimming are respectively recorded. The mice swim with the load twice every day, respectively in the morning and afternoon. The sum of the swimming times is recorded as the swimming time for one day. The average of the swimming times for the last two days is taken as a reference, and the mice are kept swimming every day until the day in which the swimming time is below 80% of the reference, and that is considered as fatigue.

### 1.3 Grouping and administration

The mice which have been identified as fatigue are divided again into five groups, i.e. model group, control group (glucose 1g/kg), ribose group (ribose 2.5 g/kg), caffeine group (caffeine 75 mg/kg), and ribose plus caffeine group (ribose 1250 mg/kg + caffeine 40 mg/kg), by counterpoint grouping method. The respective agents are given by intragastric administration with a dosage of 0.2 ml/10g, and the daily dose is given in three times. The mice are administrated with the agents 30 minutes before swimming in the morning and afternoon, respectively, and administrated again when the furs of the mice dry after swimming in the afternoon. The mice are continued to swim for three days under the prior condition as described in Section 1.2.After swimming, the respective agents are continually administered to the mice 3 times/day for 3 days, except those to be sampled immediately, , then taken as materials.

### 1.4 Experimental results

Figure 2 shows the change of the swimming time for each group calculated according to Table 1. The swimming time for each group on day 0 is set for 1, the swimming time for each group on day 1, 2 and 3 is compared to that on day 0 respectively, and thereby the change rate (%) is obtained.

The results show that after supplementation of glucose, ribose, caffeine, and ribose plus caffeine to the fatigue mice, the swimming time for caffeine group mice is shortened with the extension of training time, therefore, caffeine does not produce sustained anti-fatigue effect. The swimming time for ribose group and ribose plus caffeine group is increased with the extension of training time, namely, the anti-fatigue effect for ribose plus caffeine group is better than that for caffeine alone, and ribose plus caffeine group produces a more significant effect.

Ribose has anti-fatigue effect, as well as caffeine, but they have different action mechanisms and can be complementary with each other. Further studies on anti-fatigue effect of the composition have not been reported yet. The composition containing ribose and caffeine of the present invention may provide a more sustainable and effective anti-fatigue product and has an anti-fatigue effect of real significance.

In the state of fatigue, the administration of ribose can restore energy more quickly than the control group (i.e. glucose). The administration of caffeine alone can restore some strength in a short time but increase fatigue with the extension of time. The composition containing ribose and caffeine of the present invention can not only achieve anti-fatigue effect in a short time, but also extend the duration time of anti-fatigue effect, hence it has better anti-fatigue effect compared to caffeine.

### Example 2 Ratio study about ribose combined with caffeine

### 1. Experimental animals

Experiment animals are ICR male mice with the weight of 18-20 g, which are provided by Beijing Vital River Laboratory Animal Technology Co., certificate of conformity for: SCXK (Beijing) 2012-0001.

### 2. Drugs and reagents

D-Ribose: provided by Chengzhi Life Science and Technology Co. Ltd., batch number: 20121112.
Caffeine: provided by Chengzhi Life Science and Technology Co. Ltd..
Positive drug: CAO Kaiyong nutrient solution for men, purchased from Tianjin Kay Yong Health Products Co. Ltd., batch number: 113002. The dosage for oral administration by the human is 10 ml/piece, three pieces every day, namely 0.5 ml/kg calculated based on a body weight of 60 kg, and the dosage for the tested mice is 10 times the dosage for human, namely 5 ml/kg for mice.

### 3. Experimental methods

### 3.1 Preparation of fatigue models

A lead wire with a weight equivalent to 5% body weight of each mouse is fixed to the tail of the corresponding mouse, and the mice are placed into 40 cm×50 cm×25 cm water tanks with water tempreture of 30-33 °C and water depth of 20 cm, with ten mice per tank. The mice are made swim until the mice are fatigued (submerging in water for 3 seconds).After rest for 20 minutes, the mice swim again until the mice are fatigued. After rest 20 minutes again, the above steps are repeated. The sum of swimming time within two hours is recorded. The experimental process in the morning is performed again in the afternoon. The sum of swimming time in one day is calculated as a basis for grouping.

### 3.2 Selection of respective factor levels and administration for each group

Considering literature, routine application situation and pre-test results, D-ribose doses administered to the mice are 0.25 g, 0.5 g, 1 g/kg; and caffeine dose administered to the mice are 5 mg, 10 mg, 20 mg/kg. According to the requirements of the experimental purpose, two factors for respective three levels are selected. D-ribose groups with dosages of 0.25 g, 0.5 g, 1g/kg, caffeine groups with dosages of 5 mg, 10 mg, 20 mg/kg, and Cao Kaiyong nutrient solution groups using as control groups are set separately, 17 experimental groups are included in the present experiment in total. The corresponding agents are given by intragastric administration for three days, with a daily dose in twice and the time of administration at 10 a.m. and 10 p.m. every day. On day 4, a daily dose of each agent is given by intragastric administration once, 30 minutes after administration, a lead wire with a weight equivalent to 5% body weight of each mouse is fixed to the tail of the mouse, with the same condition as mentioned above, and the mice swim until the mice is dead. The swimming time is recorded, and the averages for each group are calculated and compared between groups.

**Table 1. Factor-level table**

| levels | factors | |
|---|---|---|
| | A D-ribose (g/kg) | B caffeine (mg/kg) |
| 1 | 0.25 | 5 |
| 2 | 0.5 | 10 |
| 3 | 1 | 20 |

### 4. Analysis Method

The average time of swimming until death for each group is calculated as a basis for data analysis, the average time for each group is compared with that for the control group and analyzed (Fig. 3). It shows from Figure 3 that, caffeine plus ribose produces different anti-fatigue effects with different proportions. The contents of 30mg for caffeine and 1.5g for ribose in food product are recommended according to the dose conversion of mice into human.

### Example 3 Effects of ribose and caffeine on recovery of high-energy phosphate substances in gastrocnemii of fatigue mice.

### 1. Establishment of fatigue models

A lead wire with a weight equivalent to 5% body weight of each mouse is fixed to the tail of the corresponding mouse, and the mice are placed into 40 cm×50 cm×25 cm water tanks with water tempreture of 30-33 °C and water depth of 20 cm, with ten mice per tank. The mice swim until the mice are fatigued (submerging in waterd for 3 seconds). After rest for 20 minutes, the mice swim again until the mice are fatigued. After rest for 20 minutes again, the above steps are repeated. Each swimming time is recorded separately until the sum of the swimming time is up to 2 hours. The experimental process in the morning is performed again in the afternoon until the sum of the swimming time is up to 2 hours.

### 2. Grouping and administration

After modeling, the gastrocnemii of 10 fatigued mice and 10 normal mice, i.e. fatigue group and normal control group, are immediately taken. The rest of the mice are randomly divided into six groups according to the swimming time on day 1, namely Cao Kaiyong group, model group, and the groups of D-ribose 1 g + caffeine 20 mg/kg (reference), the group of D-ribose 0.5 g + caffeine 10 mg/kg, the group of D-ribose 0.25 g + caffeine 5 mg/kg and the group of caffeine 20 mg/kg (reference), with the administration volume of 10 ml/kg. After modeling, the intragastric administration is performed once daily for three days, 30 minutes after the last administration, the gastrocnemii are taken as materials as stated above, and the contents of ATP, ADP, AMP and IMP in the gastrocnemii are measured by high performance liquid chromatography.

### 3. Taking and processing of the gastrocnemii

The gastrocnemii are taken from the fatigue mice and the muscle tissue samples are placed in a freezer in -80 °C and dried overnight. After weighing with a electronic scale having the sensitivity of 0.0001g, the samples are immediately placed into precooled glass grinder, with 0.4 mol/L perchloric acid solution precooled at 4 °C being added according to the solvent volume to the tissue weight ratio of 10ml/g, then quickly ground into skeletal muscle homogenates in an ice bath, mixed in a vortex for 2.0 minutes, and centrifuged at 4 °C (4000 r/min) for 15 minutes, and the supernatant is taken. The pH of the supernatant is adjusted to 6.5 with 1.0 mol/L sodium hydroxide solution precooled at 4 °C, centrifuged again at 4 °C (4000 r/min) for 15 minutes, the supernatant is taken and filtered through 0.2 µm microfiltration membrane to obtain skeletal muscle extracts, i.e. test solutions, which are stored in -80 °C for test.

### 4. Results

The results show that in the gastrocnemii of the fatigue mice after weight loading swim, the contents of high-energy phosphate substances such as ATP, ADP, AMP, etc. are decreased , while the metabolite IMP of high-energy phosphate substances is significantly increased (compared with the control group, P < 0.01). After taking a rest for 2 days, the mice of the model group have restored some the high-energy phosphate substances in the gastrocnemii. After D-ribose plus caffeine is used, ATP content in the gastrocnemii of the middle- and high-dose groups are significantly increased (compared with the model group, P < 0.05, P < 0.01). The middle- and low-dose groups of D-ribose plus caffeine also enable ADP content in the gastrocnemii of mice to be significantly increased (compared with the model group, P < 0.05, P < 0.01). Experimental results are shown in Figure 4.

### Example 4. Ribose plus coffee and the preparation thereof

The ribose plus coffee are prepared by adding ribose into instant coffee or triad coffee, with 0.01-5.0g ribose for one portion of coffee or triad coffee, and mixing evenly. For example, the ribose plus coffee can be obtained by mixing ribose 25 g with 36 g instant coffee evenly.

### Example 5. Beverage containing ribose and coffee

The formula of the beverage is that: ribose 18 g, milk powder 14 g, instant coffee 20 g, white granulated sugar 32 g, cocoa 3.9 g, stevia 0.2 g, pectin 5.4 g, soluble dietary fibers 13.8 g, vitamin C 0.5 g, essences 0.006 g and water 1000 mL.

### Reference Example 6 Food containing ribose and caffeine

1. A beverage containing ribose and caffeine may be obtained by adding 0.01-10.0% of ribose into beverage containing caffeine. Examples are given as follows.
   Formula I: ribose 8 g, caffeine 15 mg or guarana extract 200 mg (containing 10% caffeine), skimmed milk powder 20 g, white granulated sugar 18 g, fructose 5 g, vitamin C 0.6 g, aspartame 0.2 g, cyclamate 0.05 g, soluble dietary fibers 14 g, citric acid 0.9 g, sodium citrate 0.1 g, sodium benzoate 0.35 g, caramel colorant 0.06 g, vanillin 30 mg, sucrose ester 0.5 g and water 1000 mL.
   Formula II: ribose 10 g, caffeine 300 mg, taurine 4 g, magnesium chloride 0.2 g, vitamins (C, B2, niacinamide, calcium pantothenate) 0.1 g, white granulated sugar 5 g, sucralose 0.05 g, soluble dietary fibers 16 g, citric acid 1.2 g, potassium sorbate 0.3 g, food colorants 0.001 g, apple essence 0.01 g, carbon dioxide 4.8 g and water 1000 mL.
   Formula III: refreshing beverage containing ribose plus coconut and preparation thereof

   White granulated sugar is cooked in water in a jacketed kettle to form concentrated syrup with a concentration of 50%, then filtered through filter separator for use. Coconut milk is added into a mixing tank, after being diluted to a certain volume, blended with 10% filtered coconut milk, then added with citric acid solution to adjust pH to 6-7, added with 18% concentrated syrup, 1% ribose, 0.05% table salt, 0.2% emulsifier (monoglyceride), and an appropriate amount of stabilizer (xanthan gum), heated to 80°C, after high pressure homogenization, sterilized at 121 °C for 15 minutes and canned.
2. Food of energy bars (gum) containing ribose and caffeine
   Formula IV: ribose 3 g, caffeine 50 mg, taurine 2 g, vitamins (C, B2, niacinamide, calcium pantothenate) 0.1 g, white sugar 12 g, fructo-oligosaccharide 16 g, cocoa powder 8 g, cocoa butter 15 g, glucose syrup 5 g, peanut butter 5 g, soy protein 10 g, wheat flour 20 g, oat 5 g, emulsifier 0.2 g, essences 0.1 g and an appropriate amount of water.
   Formula V: ribose 3 g, caffeine 30 mg, vitamins (C, E) 0.1 g, white sugar 12 g, fructo-oligosaccharide 5 g, dietary fibers 15 g, glucose syrup 5 g, tragacanth 3 g, apple juice concentrate 1 g, emulsifier 0.2 g, essences 0.1 g, sodium chloride 0.1 g, potassium chloride 0.08 g, honey 1 g and an appropriate amount of water.
   Formula VI: ribose 3 g, caffeine 40 mg, branched-chain amino acids 1 g, white sugar 10 g, cocoa butter 24 g, cocoa powder 16 g, milk powder 5 g, glucose syrup 15 g, inulin 10 g, soy lecithin 0.2 g and essences 0.1 g.

### Reference Example 7. Candy foods containing ribose and caffeine

Formula I: ribose 100 g, caffeine 200 mg or guarana extract 2 g (containing 10% caffeine), white sugar 200 g, glucose syrup 300 g, vitamin C 3 g, citric acid 30 g, sodium citrate 3 g, apple essence 0.3 g, wolfberry extract 1 g and starch syrup 363 g.
Formula II: ribose 100 g, caffeine 200 mg or guarana extract 2 g (containing 10% caffeine), starch 25 g, starch syrup 400 g, white sugar 450 g, strawberry juice concentrate 5 g, citric acid 20 g and essences 0.2 g.
Formula III: ribose 80 g, caffeine 200 mg or guarana extract 2 g (containing 10% caffeine), xylitol 180 g, maltitol 70 g, sorbitol 30 g, mannitol 10 g, gum arabic 20 g, glucose syrup 300 g, gum base 350 g, inulin 50 g, chrysanthemum extract 1 g, pear juice concentrate 4 g, stevia 0.8 g, carnauba wax 2 g and soy lecithin 2 g.
Formula IV: ribose 6 g, white sugar 10 g, cocoa butter 34 g, cocoa powder 16 g and milk 10 g. The cocoa butter and cocoa powder are placed over a low fire and melted, added with white sugar and milk and heated until the mixture is uniform and sticky. Then ribose is added, melted, mixed evenly, and the resultant mixture is poured into a container model to obtain ribose-containing chocolate.

### Example 8. Anti-fatigue effect of ribose plus coffee

### 1. Test method

Uchida-Kraepelin psychological testing method is used to collect data. 18 healthy subjects (male 8 and female 10), aged 24-48 years old, without heart disease, diabetes and other chronic diseases, are randomly enrolled in the test. The subjects finish their lunch at 12:00, and take the coffee and ribose plus coffee respectively at 13:30, and operation test is performed at 16:00. The operation test requires the subjects to perform a continuous addition calculation of one-digit number under a certain time pressure for 15 minutes. The working status and the fatigue degree of the subjects a period of time after administration of ribose plus coffee and coffee, respectively, are judged by comparing the work amounts and the ratios of the maximum work amount to the minimum work amount. The data are processed according to statistical comparison test (SPSS) to determine the anti-fatigue effects of different beverages.

### 2. Experimental materials

Black coffee (3.6 g, caffeine content ≥50 mg/bag), ribose plus coffee (Example 3) and 1-9 random number table (108 × 16 compiled by referring to Kraepelin psychological test table).

### 3. Processing of results

The continuous addition calculation test of one-digit number under certain time pressure is performed for 15 minutes for the subjects drinking black coffee and ribose plus coffee, and the resulted data are compared in pairs and analyzed (Tables 2-4).

**Table 2 Basic descriptive statistics of 15 minutes continuous calculation test for the subjects drinking black coffee and ribose plus coffee**

| | mean | quantity | standard deviations | mean standard error |
|---|---|---|---|---|
| ribose plus coffee | 81.6124 | 11 | 5.55719 | 1.67556 |
| black coffee | 77.0273 | 11 | 6.81265 | 2.05409 |

**Table 3 Correlation coefficient and test of 15 minutes continuous calculation for the subjects drinking black coffee and ribose plus coffee**

| | quantity | Correlation | significance |
|---|---|---|---|
| ribose plus coffee & black coffee 3.8g | 11 | 0.429 | 0.188 |

**Table 4 t-test results of paired samples performing 15 minutes continuous calculation for the subjects drinking black coffee and ribose pluse coffee**

| | | Paired samples test | | | | | T value | Degree of freedom | significance (two-tailed) |
|---|---|---|---|---|---|---|---|---|---|
| | | mean | standard deviation | standard error | 95% confidence interval | | | | |
| | | | | | Low value | high value | | | |
| pairs | ribose plus coffee-black coffee | 4.58509 | 6.69330 | 2.01811 | .08847 | 9.08171 | 2.272 | 10 | .046 |

It can be seen from Table 3 that there are significant differences between the data of 15 minutes continuous addition calculation test of one-digit number under a certain time pressure for the subjects drinking ribose plus coffee and black coffee (i.e. P <0.05). The calculated amount of 15 lines continuous operations is taken as the ratio of the maximum calculated amount to the minimum calculated amount, thereby to determine the degree of fatigue. The higher the ratio is, the better the anti-fatigue effect is. It can be seen that anti-fatigue effect of drinking ribose plus coffee on the body is more significant compared to that of drinking black coffee.

Although the foregoing has been described the present invention in detail with the general description and specific embodiments, but some modifications or improvements can be made based on the present invention, which is obvious to a person skilled in the art. Therefore, these modifications or improvements without departing from the spirit of the invention fall into the scope of the invention as claimed.

### Industrial Applicability

The present invention provides a composition containing ribose and caffeine with long-lasting anti-fatigue effect, which can improve the body's energy and resist short-term and long-term fatigue in the body. The combination of caffeine and ribose can quickly refresh mind and excite spirit, while it can also supplement the energy required by the body and make the body full of physical strength and such state can be sustained. The composition is effective in improving the more tired condition and discomfort after a short time excitement in the body, which is caused by the prior product containing caffeine as main anti-fatigue ingredient, thus making the body maintain their physical strength better.

## Claims

1. A composition containing ribose and caffeine as effective ingredients, which has a sustainable anti-fatigue effect and helps the body restore the level of high-energy phosphate substances and increase energy, **characterized in that** it comprises the following components in parts by weight: 92.6-98 ribose and 2-7.4 caffeine.

2. The composition according to claim 1, **characterized in that** it further comprises one or more selected from the group consisting of milk powder or fresh milk, amino acids, sugars, sugar alcohols, cereals, cocoa, chocolate, non-saccharide and sugar alcohols sweeteners, pectin, soluble dietary fibers, salts, other carbohydrates, vitamins, minerals, carbon dioxide or other food additives;
wherein, the amino acids comprise one or more of taurine, pyruvic acid, ketoglutaric acid, arginine and citrulline; the sugars comprise one or more of glucose, sucrose or fructose; the non-saccharide and sugar alcohols sweeteners comprise one or more of stevia, aspartame, sucralose, Momordica grosvenori, glycyrrhizin, beet sweetener or saccharin sodium; the sugar alcohols comprise one or more of xylitol, erythritol, maltitol, isomalt or sorbitol; the minerals comprise one or more elements of calcium, magnesium, iron and zinc; the salts comprise one or more salts of sodium, potassium and phosphate; said other carbohydrates comprise wheat flour, inulin, oligosaccharides, starch and/or soluble or insoluble dietary fibers; said other food additives comprise one or more of essences, colorants, malic acid, citric acid, sodium citrate, sorbic acid, potassium sorbate, benzoic acid, sodium benzoate, edible gum base, sucrose esters, soy lecithin and sodium alginate; the essences and colorants are synthetic or natural; and the caffeine is derived from cocoa, coffee, guarana, tea or chocolate.

3. Non-therapeutic use of the composition according to claim 1 or 2 for preparing a healthcare product which has a sustainable anti-fatigue effect, helps the body restore the level of high-energy phosphate substances and increase energy.

## Patentansprüche

1. Zusammensetzung, die Ribose und Koffein als Wirkstoffe enthält, die eine nachhaltige Wirkung gegen Ermüdung hat und dem Körper hilft, den Pegel von hochenergetischen Phosphatsubstanzen wiederherzustellen und die Energie zu erhöhen, **dadurch gekennzeichnet, dass** sie die folgenden Komponenten in Gewichtsteilen enthält: 92,6-98 Ribose und 2-7,4 Koffein.

2. Zusammensetzung nach Anspruch 1, **dadurch gekennzeichnet, dass** sie ferner ein oder mehr Elemente umfasst, die aus der Gruppe ausgewählt werden, die besteht aus: Milchpulver oder Frischmilch, Aminosäuren, Zuckerarten, Zuckeralkoholen, Getreidearten, Kakao, Schokolade, Nicht-Saccharid- und Zuckeralkohol-Süßungsmitteln, Pektin, löslichen Nahrungsfasern, Salzen, anderen Kohlenhydraten, Vitaminen, Mineralien, Kohlendioxid oder anderen Nahrungsmittelzusatzstoffen;
wobei die Aminosäuren ein oder mehrere Elemente aus Taurin, Brenztraubensäure, Ketoglutarsäure und Citrullin; die Zucker umfassen ein oder mehr Elemente aus Glukose, Saccharose oder Fructose; die Nicht-Saccharid- und Zuckeralkohol-Süßungsmittel umfassen ein oder mehrere Elemente aus Stevia, Aspartam, Sucralose, Momordica, Glycyrrhizin, Rübenzucker oder Natriumsaccharin; die Zuckeralkohole umfassen ein oder mehrere Elemente aus Xylitol, Erythritol, Maltitol, Isomalt oder Sorbitol;
die Mineralien umfassen ein oder mehrere Elemente aus Calcium, Magnesium, Eisen und Zink; die Salze umfassen ein oder mehrere Elemente aus Natrium, Kalium und Phosphat; die anderen Kohlenhydrate umfassen Weizenmehl, Inulin, Oligosaccharide, Stärke und/oder lösliche oder unlösliche Nahrungsmittelfasern; die anderen Nahrungsmittelzusätze umfassen ein oder mehrere Elemente aus Essenzen, Farbstoffen, Apfelsäure, Zitronensäure, Natriumcitrat, Sorbinsäure, Kaliumsorbat, Benzoesäure, Natriumbenzoat, essbare Gummibase, Saccharoseester, Sojalecithin und Natriumalginat; die Essenzen und Farbstoffe sind synthetisch oder natürlich; und das Koffein ist aus Kakao, Kaffee, Guarana, Tee oder Schokolade gewonnen.

3. Nicht-therapeutische Verwendung der Zusammensetzung nach Anspruch 1 oder 2 zur Herstellung eines Gesundheitspflegeproduktes, welches einen nachhaltigen Effekt gegen Ermüdung hat, dem Körper hilft, den Pegel der hochenergetischen Phosphatsubstanzen wiederherzustellen und die Energie zu erhöhen.

## Revendications

1. Composition contenant du ribose et de la caféine en tant que principes actifs, qui a un effet antifatigue durable et aide le corps à rétablir le niveau des substances de type phosphate hautement énergétiques et à augmenter l'énergie, **caractérisée en ce qu'**elle comprend les composants suivants, en parties en poids : 92,6 à 98 de ribose et 2 à 7,4 de caféine.

2. Composition selon la revendication 1, **caractérisée en ce qu'**elle comprend en outre un ou plusieurs choisis dans le groupe constitué par le lait en poudre ou le lait frais, les acides aminés, les sucres, les alcools de sucre, les céréales, le cacao, le chocolat, les édulcorants non saccharidiques et de type alcool de sucre, la pectine, les fibres alimentaires solubles, les sels, d'autres hydrates de carbone, les vitamines, les minéraux, le dioxyde de carbone et d'autres additifs alimentaires ;
dans laquelle les acides aminés comprennent un ou plusieurs parmi la taurine, l'acide pyruvique, l'acide cétoglutarique, l'arginine et la citrulline ; les sucres comprennent un ou plusieurs parmi le glucose, le saccharose et le fructose ; les édulcorants non saccharidiques et de type alcool de sucre comprennent un ou plusieurs parmi le stevia, l'aspartame, le sucralose, Mormordica grosvenori, la glycyrrhizine, les édulcorants à base de betterave et la saccharine sodique ; les alcools de sucre comprennent un ou plusieurs parmi le xylitol, l'érythritol, le maltitol, l'isomalt et le sorbitol ; les minéraux comprennent un ou plusieurs éléments parmi le calcium, le magnésium, le fer et le zinc ; les sels comprennent un ou plusieurs de sels de sodium, de potassium, et les phosphates ; lesdits autres hydrates de carbone comprennent la farine de blé, l'inuline, les oligosaccharides, l'amidon et/ou les fibres alimentaires solubles ou insolubles ; lesdits autres additifs alimentaires comprennent un ou plusieurs parmi les essences, les colorants, l'acide malique, l'acide citrique, le citrate de sodium, l'acide sorbique, le sorbate de potassium, l'acide benzoïque, le benzoate de sodium, les bases de gomme comestibles, les esters de saccharose, la lécithine de soja et l'alginate de sodium ; les essences et colorants sont synthétiques ou naturels ; et la caféine dérive de cacao, café, guarana, thé ou chocolat.

3. Utilisation non thérapeutique de la composition selon la revendication 1 ou 2 pour préparer un produit de santé qui a un effet antifatigue durable, aide le corps à rétablir le niveau des substances de type phosphate hautement énergétiques et à augmenter l'énergie.
